# EUROPEAN PATENT SPECIFICATION

(11) **EP 3 228 266 B1**
(45) Date of publication and mention of the grant of the patent: **24.02.2021**
(21) Application number: 16164196.4
(22) Date of filing: 07.04.2016
(51) Int. Cl.: A61M 25/14, A61B 17/22, A61N 7/02, A61N 7/00, A61B 8/12, A61B 8/08, A61M 25/01, A61K 38/49, A61K 38/48

(54) **A DEVICE FOR ULTRASONIC-ACCELERATED HEMATOMA LYSIS OR THROMBOLYSIS OF INTRACEREBRAL OR INTRAVENTRICULAR HEMORRHAGES OR HEMATOMAS**
VORRICHTUNG ZUR ULTRASCHALLBESCHLEUNIGTEN HÄMATOMLYSE ODER THROMBOLYSE VON INTRAZEREBRALEN ODER INTRAVENTRIKULÄREN HÄMORRHAGIEN ODER HÄMATOMEN
DISPOSITIF À ULTRASONS ACCÉLÉRÉS DE LYSE D'HÉMATOME OU DE THROMBOLYSE D'HÉMORRAGIES OU HÉMATOMES INTRAVENTRICULAIRES OU INTRACÉRÉBRAUX

(43) Date of publication of application: 11.10.2017
(73) Proprietor: Universitätsmedizin der Johannes Gutenberg-Universität Mainz, 55131 Mainz (DE)
(72) Inventor: GIESE, Alf, 55124 Mainz (DE); KERIC, Nauren, 55131 Mainz (DE); MASOMI-BORNWASSER, Julia, 55131 Mainz (DE)
(74) Representative: Patentanwälte Dr. Keller, Schwertfeger Partnerschaft mbB

(56) References cited:
- EP-A1- 2 727 544
- US-A- 5 248 297
- US-A- 5 318 518
- US-A1- 2008 319 376
- US-A1- 2015 173 782
- US-B1- 9 211 163

## Description

The present invention concerns a device for ultrasonic-accelerated hematoma lysis or thrombolysis of intracerebral or intraventricular hemorrhages or hematomas.

The medical device according to the present invention is suitable for the treatment of brain hemorrhages.

Intracerebral hemorrhage (ICH) counts for 8 to 13 % of all strokes world-wide and results for a wide spectrum of disorders. Intracerebral hemorrhage is more likely to result in death or major disability than ischemic stroke or subarachnoid hemorrhage. Intracerebral hemorrhage usually results from bleeding associated with amyloid angiopathy, tumors, hemorrhagic conversion of ischemic stroke, dural venous sinus thrombosis, vasculitis and vascular malformations such as cavernous angiomas, arteriovenous fistulae, arteriovenous malformations, venous angiomas, and aneurysms (Qureshi et al., Spontaneous intracerebral hemorrhage. N Engl J Med. 2001b; 344:1450-60; Ruiz-Sanoval et al., Intracerebral hemorrhage in young people: analysis of risk factors, location, causes, and prognosis. Stroke: a journal of cerebral circulation. 1999; 30:537-41). ICH also results in hematomas that rupture or distort brain connections and also affect cerebral blood flow. These physical effects are generally termed the "mass effect" (Keep et al., Intracerebral haemorrhage: mechanisms of injury and therapeutic targets. Lancet Neurol. 2012; 11:720-31). For several decades there have been clinical trials of surgical clot evacuation aimed at reducing the mass effect. In a pig model of ICH, rtPA (recombinant tissue plasminogen activator) liquefied clots for aspiration, but there was also evidence of delayed edema formation (Rohde et al., Fibrinolysis therapy achieved with tissue plasminogen activator and aspiration of the liquefied clot after experimental intracerebral hemorrhage: rapid reduction in hematoma volume but intensification of delayed edema formation. J Neurosurg. 2002; 97:954-62) and an increased inflammatory response (Thiex et al., The long-term effect of recombinant tissue-plasminogen-activator (rt-PA) on edema formation in a large-animal model of intracerebral hemorrhage. Neurol Res. 2003; 25:254-62). In this model, the hematoma could be surgically removed without the use of rtPA, thereby reducing inflammation without having an effect on ICH-induced edema (Thiex et al., Minor inflammation after surgical evacuation compared with fibrinolytic therapy of experimental intracerebral hemorrhages. Neurol Res. 2005; 27:493-8).

Non-traumatic, spontaneous ICH is also associated with intraventricular hemorrhages (IVH) in 40 to 50 % of all cases. IVH is associated with mortality rates of 30 to 80 %, compared to 5 to 29 % for ICH without IVH (Hanley DF. Intraventricular hemorrhage: severity factor and treatment target in spontaneous intracerebral hemorrhage. Stroke. 2009; 40:1533-8). IVH can result in acute hydrocephalus by obstruction of the ventricular system or extraventricular compression from ICH (Lodhia et al. Hydrocephalus in a rat model of intraventricular hemorrhage. Acta neurochirurgica Supplement. 2006; 96:207-11; Zazulia AR. Hydrocephalus in ICH: what do we really know? Neurocritical care. 2008; 8:233-4). Intraventricular and intracerebral clots have been treated with rtPA (recombinant tissue plasminogen activator) by catheter-directed thrombolysis using ultrasound treatment (Newell et al., Minimally invasive evacuation of spontaneous intracerebral hemorrhage using sonothrombolysis. Journal of neurosurgery. 2001; 115:592-601).

Spontaneous intracerebral hemorrhage thus occurs in a high number of patients each year without proven effective treatment. It is estimated that the annual incidence of ICH is 10 to 30 cases per 100.000 persons per year, accounting for about 2 million strokes annually worldwide (Qureshi et al., Intracerebral haemorrahge. Lancet. 2009; 373:1632:-1644). This condition is fatal in 30 to 50 % of all occurrences, and the majority of survivors have significant motor and cognitive disabilities.

There are several different methods in order to conduct hematoma lysis or thrombolysis of intracerebral or intraventricular hemorrhages or hematomas, whereas ultrasound-induced hematoma lysis or ultrasound-accelerated thrombolysis appear to be promising approaches for treatment of such disorders. The catheter-based evacuation has been suggested as a novel surgical approach for the treatment of ICH. The safety and efficiency of ultrasound was evaluated in combination with recombinant tissue plasminogen activator (rtPA) delivered for micro catheter directly in spontaneous intraventricular (IVH) or intracerebral (ICH) hemorrhage in humans (Newell et al., Minimally invasive evacuation of spontaneous intracerebral hemorrhage using sonothrombolysis. Journal of neurosurgery. 2001; 115:592-601). All patients showed significant volume reduction in the treated hemorrhage, suggesting that lysis and drainage of spontaneous ICH and IVH with a reduction in mass effect can be accomplished rapidly and safely through sonothrombolysis using stereotactically delivered drainage and ultrasound catheters via a bur hole in the patient's skull. A ventricular drainage catheter and an ultrasound infusion micro catheter were depicted prior to placement in an ICH. A guidewire was used for insertion.

Ultrasound-accelerated thrombolysis involves the breaking down or "melting" of blood clots that can form in arteries or veins. The methodology applies ultrasound energy along with thrombolytics (e.g. tPA) in order to accelerate the process of thrombolysis. Acoustic energy creates a pressure wave that results in a disruption of fibrin and other components, and ultrasound energy also helps to create a way for the thrombolytic compound to get into the clot by loosening up the fibrin cross-links by 'acoustic streaming'. Ultrasound energy therefore alters the shape of the fibrin network, thereby dissociating the clot directly.

A combined lysis of thrombus in brain ischemia using transcranial ultrasound and systemic rtPA was used by monitoring with a 2 MHz transcranial Doppler (TCD) (Alexandrov et al., CLOTBUST: Design of a Randomized Trial of Ultrasound-Enhanced Thrombolysis for Acute Ischemic Stroke, Journal of Neuroimaging, Volume 14, Issue 2, pages 108-112, April 2004) CLOTBUST: Design of a Randomized Trial of Ultrasound-Enhanced Thrombolysis for Acute Ischemic Stroke. Journal of Neuroimaging, 14: 108-112). Image-guidance was used in order to improve catheter accuracy compared with a standard technique (Levitt et al., 2012; Image-guided cerebrospinal fluid shunting in children: catheter accuracy and shunt survival, J Neurosurg Pediatrics 10:112-117, 2012).

A common problem of catheter-based systems stems from draining hematomas which block the catheter system. Therefore, the catheter system must be frequently flushed with a syringe, which is only little efficient, and its application may also bear the danger of infection and cerebral abscess (Hoefnagel et al., Risk factors infections related to external ventricular drainage. Acta Neurochir; 150(3):209-214, 2008).

Therefore, common catheter-based systems that apply ultrasonic treatment are not optimized for efficient and protective treatment of ICH or IVH due to the fact that the ultrasonic energy also adversely affects surrounding tissue and also placement and guidance of the catheters to the ICH- or IVH-side still remains a problem.

WO 2016/007553 A describes an instrument that is specifically configured for image guided or stereotactic evacuation of intracerebral hemorrhage or other lesions. The apparatus comprises a cannula having a central channel terminating at an opening at a distal end of the cannula. The cannula is configured to be delivered through an aperture in a patient's skull for delivery to a treatment region within the cranium of the patient. A suction port is located at a proximal location of the cannula and is in fluid communication with the central channel for evacuating fluid and/or debris from the target treatment region. An irrigation port is disposed at a proximal location of the cannula, the irrigation port is in fluid communication with the distal end of the cannula for delivering fluid to the target treatment region.

US 2012/0265123 A1 describes an apparatus and a method to deliver ultrasound energy through the drain to dissolve hemorrhages and debris occluding the drain lumen and ports.

Alternative devices are described in CN103083088 A which suggests a portable positioning device for intracerebral cathetering for treatment of cerebral hemorrhages. Positioning of the device is accomplished by computed tomography (CT) such that the device can accurately reach the puncture site.

CN201379823 Y describes a device for draining a hematoma of hypertensive cerebral hemorrhage (HCH) which uses two rows of drainage holes that are in fluid communication with an intracerebral hematoma drainage silicone tube. A metal lead pin is used to be inserted into the intracerebral hematoma drainage silicone tube.

JP2006043200 A describes a support system that is equipped with an image acquiring device for acquiring a brain image of a patient, a cerebral region detection part for determining a cerebral region from the brain image and a wrinkle range detection part for detecting a region of the brain wrinkle and an uneven region of the brain by determining the difference between a convex closure in the cerebral region and the brain region for the treatment of ICH. A blood vessel removing part is used for removing a blood vessel region from the wrinkle region.

US 2008/319376 A1, US 5 318 518 A, EP 2 727 544 A1, US 9 211 163 B1, US 2015/173782 A1, and US 5 248 297 A represent additional relevant prior art.

The known catheter-based devices and methods are laborious and expensive and often result in a blockade of the catheter, which affects a rapid pressure release of the brain and drainage of toxic metabolites from the hematoma. Thrombolytic substances such as rtPA will become less efficient in mature hematomas. Therefore, a rapid drainage of fluid and debris is crucial for the success for hematoma lysis or thrombolysis of ICH or IVH. On the other side, self-flushing systems suffer from the problem that they cannot be positioned or navigated to the puncture site efficiently. In addition, although ultrasonic treatment has been proven to increase efficiency of a lysis therapy, transcranial ultrasonic has also been associated with increased bleedings. None of the known devices or methods is optimized to remain intracerebrally and intraventricularly for a prolonged period of time to mediate drainage or as monitoring system.

In studies that use ultrasonic-accelerated hematoma lysis or thrombolysis of artery occlusion, treatment of strokes occurs unselectively and/or affects healthy areas within the tissue, resulting in an increased risk of bleedings, tissue damages, blood vessel damages, in particular in areas of the cerebral infarct (Nedelmann et al., Therapeutic ultrasound of acute cerebral artery occlusion. Der Nervenarzt. 2008;79(12):1399-400, 402-6; Alexandrov AV et al., Design of a randomized trial of ultrasound-enhanced thrombolysis for acute ischemic stroke. J Neuroimaging 2004; 14(2): 108-112).

It is therefore the object of the present invention to provide an improved medical device for treatment of intracerebral or intraventricular hemorrhages or hematomas that minimizes negative side effects that are associated with the application of ultrasonic on tissues, blood vessels or hematomas.

This object is solved by a medical device comprising the features of claim 1.

The device of the present invention combines an ultrasonic-mediated imaging system, pressure sensoring means in the application of ultrasonic in the treatment of intracerebral or intraventricular hemorrhages or hematomas (ICH or IVH). The device comprises a body that accommodates different separated compartments or lumens consisting of a flushing catheter for flushing fluid and/or pharmaceutically active substances into the intracerebral or intraventricular hemorrhages or hematomas, a drainage catheter for draining fluid from the intracerebral or intraventricular hemorrhages or hematomas, an ultrasonic probe duct and a pressure sensor duct. The ultrasonic probe duct integrates either an ultrasonic probe or a stiletto (or stylet), and the pressure sensor duct integrates a pressure sensor to promote treatment of ICH and IVH. The stylet enables quick freehand navigated placements of the ventricular catheter.

The device according to the present invention allows efficient drainage of fluid and debris from the ICH or IVH site and a site-directed application of thrombolytics such as rtPA over the lumen of the flushing catheter. At the same time, the pressure sensor arranged in the present sensor duct allows monitoring of the intracranial pressure during flushing and lysis. In a further lumen of the body which forms the ultrasonic probe duct, an endosonographic probe, preferably a 10F endosonographic probe, is integrated for B-scan imaging, Doppler and duplex measurements. The endosonographic probe provides high-definition imaging of intracranial structures and allows guiding of the device to the ICH- or IVH-sites. Ultrasonic-guided imaging allows analysis of blood flow and the lysis of hematomas in intracerebral and intracranial vessels. The endosonographic probe thus allows imaging to monitor evolution of ICH and eventually a resulting brain edema in real time. In the context of the present invention, the endosonographic probe is thus used both for imaging and treatment of ICH and IVH and allows monitoring of the progress of hematoma lysis.

Endosonography using the ultrasonic probe of the invention further allows the identification of bleedings, the presence of edemas and eventual side effects on surrounding brain components including, but not limited to compression of cerebral ventricles or a displacement of the cerebral midline. Ultrasonic imaging also replaces parts of CCT (Cranial Computed Tomography) diagnostics and can be applied to all sites of intracerebral or intraventricular hemorrhages or hematomas over a prolonged period of time (e.g. up to 2 - 3 weeks). This significantly reduces CCT-dependent radiation impact on the patient.

In a preferred embodiment, the outer wall of the ultrasonic probe duct in the lower part portion of the body further comprises a membrane, which is permeable for ultrasound. The membrane can be integrated fully or partially in the catheter wall to emit ultrasonic energy. Furthermore, it is preferred that the endosonographic probe is rotatable within the ultrasonic probe duct. If the ultrasonic-permeable membrane is integrated only partially in the ultrasonic duct, it may be preferable to rotate the whole catheter in order to achieve an unidirectional emission of ultrasonic energy.

The lumen of the ultrasonic probe duct not only allows integration of the endosonographic imaging and treatment probe, but also of a stiletto that can be interchangeably guided within the ultrasonic probe duct. The stiletto or stylet is preferably inserted at the time of implantation of the device of the invention. The stiletto provides a higher stiffness for puncture through the cerebral tissue and the hematoma and therefore helps in guiding the device for neuronavigation for an image-guided placement of the catheter system to the center of ICH or IVH, at the same time conserving surrounding cerebral tissue. Upon placement of the catheter system at the target site, the stiletto is replaced by an ultrasonic probe.

Neuro-navigated placement of the device is mediated through a puncture site or hole in the skull to the center of the hematoma. In a preferred embodiment, the outside part of the body of the device of the present invention comprises a connector for cranial fixation, which is adapted to allow angular adjustment of the body both in longitudinal and/or transverse direction. The connector is preferably provided as a connector block that integrates the body with compartments or lumens of the device of the present invention. The connector allows a free movement at different angles, an adjustment of the catheter trajectory within the port system and an entanglement of the body relative to the port. After adjustment of the catheter, the connector can be locked into position.

The endosonographic probe further allows variation of the ultrasonic frequency in order to limit depth of penetration and fast tissue exposition for ultrasonic energy. A minimization of penetration depth to the target sites of ICH and IVH reduces complications and side effects such as bleedings. At the same time, the knowledge about the exact position and the provision of coordinates provide valuable information on the form and volume of bleedings. Ultrasonic ICH or IVH can be adjusted in order to influence sonographic peak pressure and penetration depth of sonic energy. All these measures contribute to a minimization of focus energy specifically to the location of the intracerebral or intraventricular hemorrhages or hematomas and a protection of surrounding healthy tissues.

Preferably, hematoma lysis is carried out at ultrasonic frequencies between 5,5 to 10 MHz. A preferred frequency of 10 MHz showed the safest modalities (MI (mechanical index) of 0.55) and the best imaging. Ultrasonic treatment and the additional provision of pharmaceutically active substances such as rtPA via the flushing catheter into the intracerebral or intraventricular hemorrhages or hematomas are preferred in order to dissociate associated clots. The device of the present invention allows high-detailed multimodular intracranial endosonographic imaging in real time and simultaneous flushing of pharmaceutically active substances into the ICH or IVH over prolonged periods of time, wherein at the same intracerebral or intraventricular fluid from the hemorrhages or hematomas is drained by the lumen of a separate drainage catheter which is part of the body. Intracerebral or intraventricular pressure is measured during the procedure by a pressure sensor that is integrated within a pressure sensor duct, and which is also part of the body of the device of the present invention.

The flushing catheter, the drainage catheter, the ultrasonic probe duct and the pressure sensor duct of the device of the present invention are essential parts of the basic body and thus, these elements are integrated in a single unit that allows better handling of the device during surgery and treatment. As part of the unit, the flushing catheter, the drainage catheter, the ultrasonic probe duct and/or the pressure sensor duct are arranged in proximity to each other and all compartments or lumens are at least sectionally isolated by fluid-tight catheter walls. The ultrasonic probe duct is configured to integrate the endosonographic probe or, if required, the stiletto for implantation.

In order to improve the clinical application of the device and the supply/drainage of substances to/from the catheters, the upper part of the flushing catheter and/or drainage catheter laterally projects from the body in bended form. The lower part of the flushing catheter disembogues into the drainage catheter in that the foot end of the catheter is permeably connected by means of one or more penetrations. For draining fluid from the ICH or IVH, the drainage catheter at the lower part of the catheter comprises apertures at the outer wall that allow uptake of the fluid and debris from the hematoma into the catheter lumen for subsequent transport to a collecting container. In the upper part of the body of the device, the surrounding catheter wall of the drainage is fully closed and does not contain apertures to allow drainage of fluid or debris to the outlet of the drainage catheter. For accurate measurements, the pressure sensor is preferably arranged opposite to the flushing catheter within the body of the device. Preferably, the pressure sensor duct is arranged in the outer diameter of the body. The ultrasonic probe duct is arranged oppositely to the drainage catheter, wherein at least a section of the flushing catheter is arranged between the drainage catheter and the ultrasonic probe duct.

The stiletto (stylet) that is guided during implantation of the device within the ultrasonic probe duct is preferably equipped with a marker of neuro navigation. Preferred markers of neuro navigations are disposable marker spheres, preferably coated with IR-light, retro-reflective foil, passive markers, or multi-modality fiducial markers. In a preferred embodiment, the neuro navigation markers are passive marker plates incorporated in the stylet. The marker geometry is automatically recognized by a navigation camera for quick calibration. Preferred reflective markers are plastic spheres with a glass-grain coating to reflect infrared light emitted by one or more cameras. The reflection is detected by detectors arranged around the cameras.

The pressure sensor is part of a pole with a diameter which allows guidance of the pole within the pressure probe duct. The pressure sensor is arranged at the cone end of the pole, i.e. at the lower part of the body.

A method, not according to the invention, for treatment of intracerebral or intraventricular hemorrhages or hematomas (ICH or IVH) via ultrasound-accelerated hematoma lysis thrombolysis applies a device (catheter system) of the invention that comprises a body, which accommodates a number of separate compartments or lumens. Not according to the invention, the disclosure also concerns the use of such a device for treatment of intracerebral or intraventricular hemorrhages or hematomas, wherein the application of a device comprises the following steps:
- flushing fluid and/or pharmaceutically active substances by means of a flushing catheter which forms an integral part of the body of the catheter system into the intracerebral or intraventricular hemorrhages or hematomas;
- draining fluid from the intracerebral or intraventricular hemorrhages or hematomas by means of a drainage catheter which forms an integral part of the body of the catheter system;
- guiding a stiletto within the ultrasonic probe duct which is arranged next to the flushing catheter and the drainage catheter in the body of the catheter system for intracranial placement of the catheter system;
- changing the stiletto against an endosonographic probe and guiding the endosonographic probe within the ultrasonic probe duct, and
- inserting a pressure probe into a pressure sensor duct of the body of the catheter system for monitoring intracranial pressure during flushing and hematoma lysis.

In a preferred embodiment, the pharmaceutically active substances are thrombolytics such as recombinant tissue plasminogen activator (rtPA), streptokinase, p-anisoylated lys-plasminogen-streptokinase activator complex, urokinase, and prourokinase. The tissue plasminogen activator compounds include Alteplase (tPA), Reteplase (sometimes called rPA), and Tenecteplase. The endosonographic probe is used both for ultrasonic lysis and real time imaging of the ICH or IVH.

The device of the present invention allows a more efficient ultrasonic-mediated ICH or IVH lysis, thereby protecting surrounding tissue. Hematoma drainage, catheter flushing and rtPA application are carried out via different separate catheter lumens within the body of the device, wherein a separate pressure sensor duct bears a pressure sensor in the lower part of the body in order to monitor intracranial pressure during flushing and lysis.

One advantage of the device of the present invention is that it can be left in the cerebrum following lysis therapy to monitor intracranial pressure and for drainage fluid from the ICH or IVH site over the drainage catheter as integral part of the device of the invention. Ultrasonic-mediated imaging and ultrasonic-accelerated hematoma lysis or thrombolysis allow a real-time imaging and monitoring of the hematoma lysis process. The combination of navigation-mediated and angle-controlled integration of the device into the ICH or IVH and the implementation of a removable stiletto are required for accurate placing of the catheter system in the center of the ICH or IVH. An additional connector allows an angular adjustment of the body in longitudinal and/or transverse direction; the device thereby can be placed in optimal orientation relative to the hematoma. This allows an efficient draining of the trajectory and simultaneous protection of important tissues and brain areas.

The present invention is illustrated in more detail in the accompanying figures.

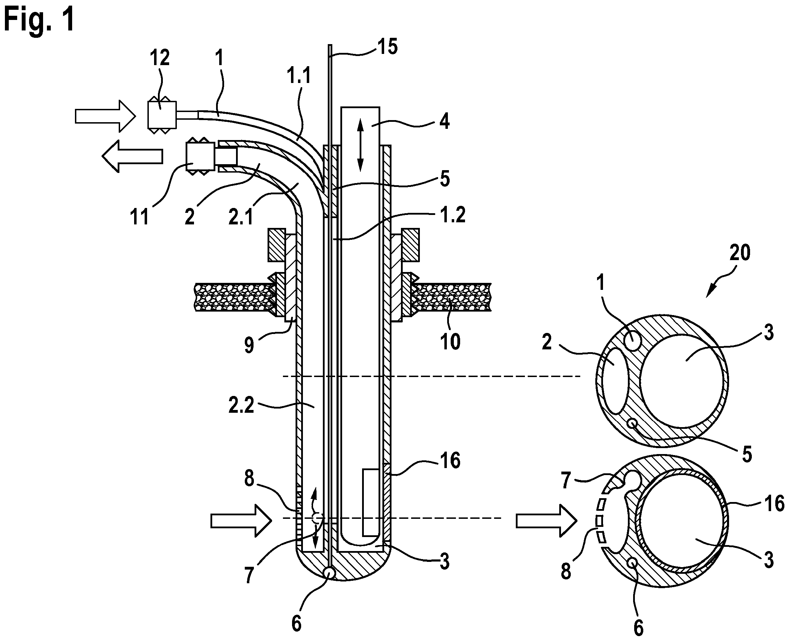

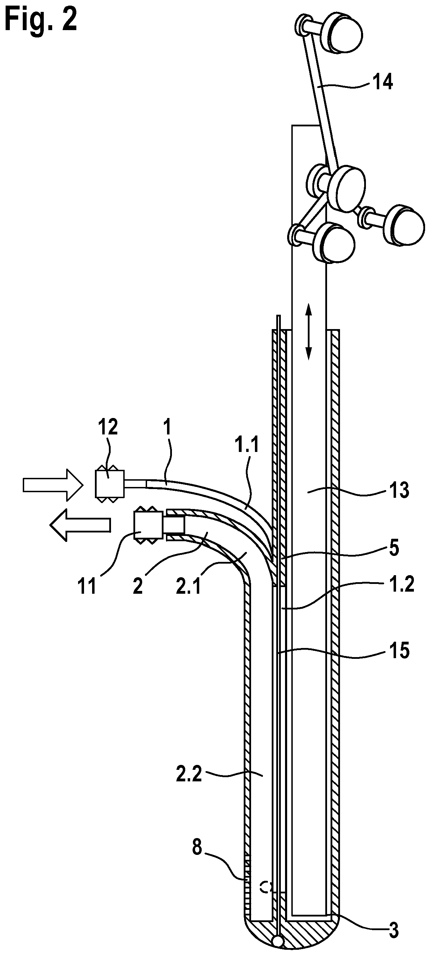

Fig. 1 shows an embodiment of the sonothrombolytic catheter system according to the present invention. The device is comprised of a body 20 that accommodates different compartments or lumens that are arranged in proximity to each other and are separated from each other by respective walls. Basically, the device of the invention contains several catheters forming a catheter system that includes different functions. The first compartment or lumen is part of a flushing catheter 1 for flushing fluid and/or pharmaceutically active substances into the intracerebral or intraventricular hemorrhages or hematomas. Preferably, rtPA or other thrombolytic substances are applied to the site of ICH or IVH by means of the flushing catheter 1. The body 20 further accommodates a compartment or lumen of a drainage catheter 2 for draining fluid from the ICH or IVH. The body 20 also comprises a compartment or lumen of an ultrasonic probe duct 3 and a separate pressure sensor duct 5. The lumen of the pressure sensor duct 5 integrates a pressure sensor 6, preferably at the cone end of a pole 15 for measurement and analysis of intracranial pressures during implantation and treatment of ICH or IVH. The ultrasonic probe duct 3 both comprises either an endosonographic probe 4 (Fig. 1) or a stiletto 13 (Fig. 2) that can be interchangeably guided within the ultrasonic probe duct 3. The upper portion 1.1 of the flushing catheter 1 and/or the upper portion 2.1 of the drainage catheter 2 laterally project from the body 20 in bended form. The lower portion 1.2 of the flushing catheter 1 and/or the lower portion 2.2 of the drainage catheter 2 are provided in straight form.

In the lower part of the body 20, the outer wall of the drainage catheter 2 comprises one or more apertures 8. Fluid or debris is flushed from the site of ICH or IVH through the catheter lumen to the upper end of the drainage catheter 2. The upper portion 1.1 of the flushing catheter 1 and/or the upper portion 2.1 of the drainage catheter 2 is equipped with adapters 11 or ports 12, respectively.

In the lower part of the body 20, the lumen of the flushing catheter 1 is penetrated such that flushing fluid from the flushing catheter 1 flows into the lower part portion 2.2 of the drainage catheter 2 through one or more penetrations 7.

The body 20 also comprises a connector 9 for cranial fixation, which is adapted to allow an angular adjustment of the body 20 in longitudinal and/or transverse direction. The connector 9 is integrated into a hole of a skull 10.

In a preferred embodiment, the circular lateral wall of the ultrasonic probe duct 3 further comprises a membrane 16, which is permeable for ultrasound. Furthermore, it is preferred that the endosonographic probe 4 is rotatable within the ultrasonic probe duct 3.

Fig. 2 shows a replacement of the endosonographic probe 4 by a stiletto 13 which is required for implantation into the ICH or IVH. The stiletto 13 provides stiffness for the implantation of the catheter system and also may comprise additional markers of neuronavigation 14 at the upper end, such as reflective marker spheres.

## Claims

1. A device for ultrasonic-accelerated hematoma lysis or thrombolysis of intracerebral or intraventricular hemorrhages or hematomas, comprising an endosonographic probe (4), a stiletto (13), and a body (20) which accommodates different compartments or lumens that are arranged in proximity to each other, wherein in longitudinal extension at least upper part proximal sections of the compartments or lumens are isolated by walls, consisting of
- a flushing catheter (1) for flushing fluid and/or pharmaceutically active substances into the intracerebral or intraventricular hemorrhages or hematomas, whereby a first compartment or lumen of the body (20) is part of the flushing catheter (1),
- a drainage catheter (2) for draining fluid from the intracerebral or intraventricular hemorrhages or hematomas, whereby a second compartment or lumen of the body (20) is part of the drainage catheter (2),
- an ultrasonic probe duct (3), and
- a pressure sensor duct (5) that integrates a pressure sensor (6), whereby
- the ultrasonic probe duct (3) integrates either the endosonographic probe (4) or the stiletto (13) that are interchangeably guided within the ultrasonic probe duct (3),
- the upper part proximal portion (1.1) of the flushing catheter (1) and/or upper part proximal portion (2.1) of the drainage catheter (2) laterally projects from the body (20) in bended form,
**characterized in that**
- in the lower distal parts (1.2, 2.2) of the body (20), the drainage catheter (2) and the flushing catheter (1) are permeably connected to each other by one or more penetrations (7),
- the pressure sensor (6) is part of a pole having a diameter which allows guidance in the pressure sensor duct (5), wherein the pressure sensor (6) is arranged at a cone end of the pole.

2. The device according to claim 1, wherein in the lower distal part (2.2) of the body (20), the outer wall of the drainage catheter (2) comprises apertures (8).

3. The device according to any one of claims 1 to 2, wherein the ultrasonic probe duct (3) is arranged oppositely to the drainage catheter (2), and wherein at least a section of the flushing catheter (1) is arranged between the drainage catheter (2) and the ultrasonic probe duct (3).

4. The device according to any one of claims 1 to 3, wherein the pressure sensor duct (5) that integrates the pressure sensor (6) is arranged at the outer part of the body (20).

5. The device according to any one of claims 1 to 4, wherein the outside part of the body (20) comprises a connector (9) for cranial fixation, which is adapted to allow an angular adjustment of the body (20) in longitudinal and/or transverse direction.

6. The device according to any one of claims 1 to 5, wherein the outer wall of the ultrasonic probe duct (3) further comprises a membrane 16 at the lower part (2.2) portion of the body (20), which is permeable for ultrasonic energy.

7. The device according to any one of claims 1 to 6, wherein the endosonographic probe (4) integrated in the ultrasonic probe duct (3) emits at frequencies between 5.5 and 10 MHz, preferably 10 MHz.

8. The device according to any one of claims 1 to 6, wherein the stiletto (13) is equipped with a marker of neuronavigation (14).

9. The device according to any one of claims 1 to 8, wherein the pharmaceutically active substances are thrombolytics such as recombinant tissue plasminogen activator (rtPA), streptokinase, p-anisoylated lys-plasminogen-streptokinase activator complex, urokinase, and prourokinase.

## Patentansprüche

1. Vorrichtung zur ultraschallbeschleunigten Hämatomlyse oder Thrombolyse von intrazerebralen oder intraventrikulären Blutungen oder Hämatomen, umfassend eine Endosonographiesonde (4), ein Stilett (13) und einen Körper (20), der verschiedene Kompartimente oder Lumen aufnimmt, die nahe zueinander angeordnet sind, wobei in Längserstreckung zumindest obere, proximale Abschnitte der Kompartimente oder Lumen durch Wände getrennt sind, bestehend aus
- einem Spülkatheter (1) zum Einspülen von Flüssigkeit und/oder pharmazeutisch aktiven Substanzen in die intrazerebralen oder intraventrikulären Blutungen oder Hämatome, wobei ein erstes Kompartiment oder Lumen des Körpers (20) Teil des Spülkatheters (1) ist,
- einem Drainagekatheter (2) zum Ableiten von Flüssigkeit aus den intrazerebralen oder intraventrikulären Blutungen oder Hämatomen, wobei ein zweites Kompartiment oder Lumen des Körpers (20) Teil des Drainagekatheters (2) ist,
- einem Ultraschallsondenkanal (3) und
- einem Drucksensorkanal (5) mit einem integrierten Drucksensor (6), wobei
- in den Ultraschallsondenkanal (3) entweder die endosonographische Sonde (4) oder das Stilett (13) integriert ist, die austauschbar im Ultraschallsondenkanal (3) geführt werden,
- der obere proximale Teil (1.1) des Spülkatheters (1) und/oder der obere proximale Teil (2.1) des Drainagekatheters (2) in gebogener Form seitlich aus dem Körper (20) herausragt,
**dadurch gekennzeichnet, dass**
- in den unteren distalen Teilen (1.2, 2.2) des Körpers (20) der Drainagekatheter (2) und der Spülkatheter (1) durch einen oder mehrere Durchlässe (7) permeabel miteinander verbunden sind,
- der Drucksensor (6) Teil eines Stabes ist, der einen Durchmesser aufweist, der die Führung im Drucksensorkanal (5) ermöglicht, wobei der Drucksensor (6) an einem konischen Ende des Stabs angeordnet ist.

2. Vorrichtung nach Anspruch 1, wobei im unteren distalen Teil (2.2) des Körpers (20) die Außenwand des Drainagekatheters (2) Öffnungen (8) umfasst.

3. Vorrichtung nach einem der Ansprüche 1 bis 2, wobei der Ultraschallsondenkanal (3) dem Drainagekatheter (2) gegenüberliegend angeordnet ist, und wobei wenigstens ein Abschnitt des Spülkatheters (1) zwischen dem Drainagekatheter (2) und dem Ultraschallsondenkanal (3) angeordnet ist.

4. Vorrichtung nach einem der Ansprüche 1 bis 3, wobei der Drucksensorkanal (5), in dem der Drucksensor (6) integriert ist, am äußeren Teil des Körpers (20) angeordnet ist.

5. Vorrichtung nach einem der Ansprüche 1 bis 4, wobei der äußere Teil des Körpers (20) einen Anschluss (9) zur kranialen Fixierung aufweist, der geeignet ist, eine Winkelverstellung des Körpers (20) in Längs- und/oder Querrichtung zu ermöglichen.

6. Vorrichtung nach einem der Ansprüche 1 bis 5, wobei die Außenwand des Ultraschallsondenkanals (3) am unteren Teil (2.2) des Körpers (20) ferner eine Membran (16) aufweist, die für Ultraschallenergie durchlässig ist.

7. Vorrichtung nach einem der Ansprüche 1 bis 6, wobei die im Ultraschallsondenkanal (3) integrierte Endosonographiesonde (4) bei Frequenzen zwischen 5,5 und 10 MHz, vorzugsweise 10 MHz, emittiert.

8. Vorrichtung nach einem der Ansprüche 1 bis 6, wobei das Stilett (13) mit einem Marker der Neuronavigation (14) ausgestattet ist.

9. Vorrichtung nach einem der Ansprüche 1 bis 8, wobei die pharmazeutisch aktiven Substanzen Thrombolytika sind, wie rekombinanter Gewebeplasminogenaktivator (rtPA), Streptokinase, p-anisoylierter Lys-Plasminogen-Streptokinase-Aktivator-Komplex, Urokinase und Prourokinase.

## Revendications

1. Dispositif pour la lyse d'hématomes ou la thrombolyse d'hémorragies ou d'hématomes intracérébrales/intracérébraux ou intraventriculaires accélérée par ultrasons, comprenant : une sonde endosonographique (4), un stylet (13) et un corps (20) qui loge différents compartiments ou différentes lumières qui sont agencé(e)s à proximité les un(e)s des autres, dans lequel, selon une extension longitudinale, au moins des sections proximales de partie supérieure des compartiments ou des lumières sont isolées par des parois, constitué par :
- un cathéter de rinçage (1) pour procéder à un rinçage au moyen d'un fluide et/ou de substances pharmaceutiquement actives à l'intérieur des hémorragies ou hématomes intracérébrales/intracérébraux ou intraventriculaires, d'où il résulte qu'un premier compartiment ou qu'une première lumière du corps (20) constitue une partie du cathéter de rinçage (1) ;
- un cathéter de drainage (2) pour drainer le fluide hors des hémorragies ou hématomes intracérébrales/intracérébraux ou intraventriculaires, d'où il résulte qu'un second compartiment ou qu'une seconde lumière du corps (20) constitue une partie du cathéter de drainage (2) ;
- un conduit de sonde à ultrasons (3) ; et
- un conduit de capteur de pression (5) qui intègre un capteur de pression (6), d'où il résulte que :
- le conduit de sonde à ultrasons (3) intègre soit la sonde endosonographique (4), soit le stylet (13), lesquels sont guidés de façon interchangeable à l'intérieur du conduit de sonde à ultrasons (3) ;
- la section proximale de partie supérieure (1.1) du cathéter de rinçage (1) et/ou la section proximale de partie supérieure (2.1) du cathéter de drainage (2) font/fait saillie latéralement depuis le corps (20) selon une forme fléchie ;
**caractérisé en ce que** :
- dans les parties distales inférieures (1.2, 2.2) du corps (20), le cathéter de drainage (2) et le cathéter de rinçage (1) sont connectés l'un à l'autre de façon perméable au moyen d'un ou de plusieurs passage(s) (7) ; et
- le capteur de pression (6) est une partie d'une tige qui présente un diamètre qui permet le guidage à l'intérieur du conduit de capteur de pression (5), dans lequel le capteur de pression (6) est agencé au niveau d'une extrémité en forme de cône de la tige.

2. Dispositif selon la revendication 1, dans lequel, à l'intérieur de la section de partie distale inférieure (2.2) du corps (20), la paroi externe du cathéter de drainage (2) comprend des ouvertures (8).

3. Dispositif selon l'une quelconque des revendications 1 et 2, dans lequel le conduit de sonde à ultrasons (3) est agencé à l'opposé du cathéter de drainage (2), et dans lequel au moins une section du cathéter de rinçage (1) est agencée entre le cathéter de drainage (2) et le conduit de sonde à ultrasons (3).

4. Dispositif selon l'une quelconque des revendications 1 à 3, dans lequel le conduit de capteur de pression (5) qui intègre le capteur de pression (6) est agencé au niveau de la partie externe du corps (20).

5. Dispositif selon l'une quelconque des revendications 1 à 4, dans lequel la partie extérieure du corps (20) comprend un connecteur (9) pour une fixation crânienne, lequel connecteur est adapté pour permettre un réglage angulaire du corps (20) dans des/une direction(s) longitudinale et/ou transversale.

6. Dispositif selon l'une quelconque des revendications 1 à 5, dans lequel la paroi externe du conduit de sonde à ultrasons (3) comprend en outre une membrane (16) au niveau de la section de partie distale inférieure (2.2) du corps (20), laquelle membrane est perméable à l'énergie ultrasonore.

7. Dispositif selon l'une quelconque des revendications 1 à 6, dans lequel la sonde endosonographique (4) qui est intégrée à l'intérieur du conduit de sonde à ultrasons (3) émet à des fréquences entre 5,5 MHz et 10 MHz, de préférence à 10 MHz.

8. Dispositif selon l'une quelconque des revendications 1 à 6, dans lequel le stylet (13) est muni d'un marqueur de neuronavigation (14).

9. Dispositif selon l'une quelconque des revendications 1 à 8, dans lequel les substances pharmaceutiquement actives sont des thrombolytiques tels qu'un activateur de plasminogène tissulaire recombinant (rtPA), la streptokinase, un complexe activateur de lys-plasminogène-streptokinase p-anisoylé, l'urokinase et la prourokinase.
